# EUROPÄISCHE PATENTSCHRIFT

(11) **EP 2 356 432 B1**
(45) Veröffentlichungstag und Bekanntmachung des Hinweises auf die Patenterteilung: **08.11.2017**
(21) Anmeldenummer: 09805673.2
(22) Anmeldetag: 10.11.2009
(51) Int. Cl.: G01N 21/85, G01N 33/22, G01N 33/28

(54) **SENSORANORDNUNG**
SENSOR ARRANGEMENT
ENSEMBLE CAPTEUR

(30) Priorität: 10.11.2008 DE 102008056559
(43) Veröffentlichungstag der Anmeldung: 17.08.2011
(73) Patentinhaber: FAUDI Aviation GmbH, 35260 Stadtallendorf (DE)
(72) Erfinder: WILDSCHÜTZ, Marcus, 41469 Neuss (DE); ADEN, Matthias, 64668 Rimbach (DE)
(74) Vertreter: Patentanwälte Olbricht Buchhold Keulertz
(86) Internationale Anmeldenummer: PCT/DE2009/001584
(87) Internationale Veröffentlichungsnummer: WO 2010/051806

(56) Entgegenhaltungen:
- EP-A1- 0 423 367
- US-A- 3 906 241
- US-A- 5 678 751
- US-A1- 2007 103 690

## Beschreibung

Die vorliegende Erfindung betrifft gemäß dem Oberbegriff von Anspruch 1 eine optische Sensoranordnung zur Detektion eines ersten flüssigen Mediums in einem zweiten flüssigen Medium mittels Reflexion eines emittierten Lichtstrahls einer Wellenlänge an einer Reflexionsfläche, mit nur einer Lichtquelle sowie einem zugehörigen Empfänger.

Es ist bekannt, dass bei Herstellung, Lagerung und Transport von flüssigen Treibstoffen auf Basis von Kohlenwasserstoffen, wie z. B. Kerosin, eine Kontamination mit Wasser durch den Kontakt mit feuchter Luft, aber auch mit Lagerbehältern und Transportleitungen erfolgt. Kerosin ist hygroskopisch und zieht damit Wasser an. Wenn die Löslichkeitsgrenze überschritten wird, bilden sich zunächst Emulsionen sehr feinteiliger Wassertröpfchen in der Treibstoffbasis. Diese feinteiligen Wassertröpfchen werden aufgrund der Hygroskopizität größer und können sich dann als geschlossene Sumpfphase auf dem Boden absetzen. In den Treibstoffen ist daher häufig Wasser (freies sowie gebundenes) vorhanden. Aus Sicherheitsgründen ist es notwendig, dieses Wasser mittels regelmäßiger Reinigung zu entfernen, da ansonsten die betriebenen Motoren beschädigt werden können. Die Abscheidung von suspendiertem Wasser kann z. B. mit Hilfe von Koaleszenzabscheidern erfolgen.

In neuerer Zeit sind insbesondere bestimmte Treibstoffzusätze problematisch, da sie häufig das Abscheiden von Wasser aus den Treibstoffen erschweren. Gelegentlich führt auch menschliches Versagen zu einer versehentlichen Befüllung von Tankanlagen mit Gemischen von Treibstoffen und Wasser oder sogar reinem Wasser. Speziell für die Luftfahrtindustrie gelten aus verständlichen Gründen ganz besonders hohe Sicherheitsanforderungen. Daher bestehen Bestrebungen, die gesamte Lieferkette der Treibstoffe von der Raffinerie bis zur Betankung zu überwachen und dabei sowohl den Gehalt an freiem Wasser als auch die Anwesenheit von Wasser anstelle von Treibstoff schnell zu erfassen und damit rechtzeitig vor Gefahren zu warnen und diese auszuschalten.

Im Stand der Technik gibt es dazu eine Reihe von ermittelten Lösungen. Die britischen Patente 1,460,623 sowie 1,554,309 bedienen sich dazu Vorrichtungen, die eine Messkammer benötigen. Problematisch dabei ist, dass entweder bei einer kontinuierlichen Messung nur qualitative Aussagen getroffen werden können (GB 1,460,623), bzw. quantitative Ergebnisse nur bei einer Intervallmessung detektiert werden (GB 1,554,309). Ein Detektor der kontinuierlich und quantitativ freies Wasser in Treibstoffen sowie gleichzeitig die Anwesenheit von reinem Wasser in Behältern und Leitungen bestimmen kann, ist aus diesen Schriften nicht bekannt.

US 3,906,241 A beschreibt drei parallele Fiberoptikkanäle, deren Enden in einer gemeinsamen Ebene liegen, die senkrecht zur Zylinderachse der Optiken liegt. Ein von einem ersten Fiberoptikkanal emittierter Lichtstrahl gelangt über zwei gegenüber den Enden der Fiberoptikkanäle angeordnete Reflexionsflächen zum zweiten Fiberoptikkanal. Die Reflexionsflächen sind hierfür symmetrisch in Form einer Pyramide mit einem Flankenwinkel von 45° ausgerichtet. Auf dem Weg vom ersten zum zweiten Fiberoptikkanal kann in einem dabei durchdrungenen Medium Streulicht entstehen. Um dieses Streulicht zu detektieren sieht US 3,906,241 A den dritten Fiberoptikkanal vor, der mit einem Empfänger verbunden ist.

US 2007/103690 A offenbart einen spektrometrischen Sensor, aufweisend eine äußere Sensorfläche, die eine Aussparung aufweist, wenigstens eine Emissionsstelle für Licht in der äußeren Sensorfläche, wobei wenigstens eine zur Aussparung zeigt, wenigstens eine Sammelstelle für von den Emissionsstellen emittiertes Licht in der äußeren Sensorfläche, wobei wenigstens eine der Sammelstellen Licht von der Aussparung aufnimmt, und wobei die Sammelstellen wenigstens zwei von drei der folgenden Merkmale aufweisen:
(a) eine in der Aussparung positionierte Transmissionssammelstelle, die Transmissionslicht von der anderen Seite der Aussparung empfängt,
(b) eine Transflektionssammelstelle in der Aussparung, die transmittiertes und auf der gegenüberliegenden Seite der Aussparung reflektiertes Licht einer Emissionsstelle empfängt,
(c) eine Reflektionssammelstelle, die in der äußeren Sensorfläche angeordnet ist und Licht einer der Emissionsstellen empfängt, wobei das reflektierte Licht weder an einer gegenüberliegenden Seite der Emissionsstelle reflektiert noch emittiert ist.
Die Grenzflächen der Sammel- und Emissionsstellen sind dabei senkrecht zur Längsachse der lichtleitenden Optikkanäle ausgebildet. Emittiertes Licht wird somit entweder gemäß (c) überhaupt nicht zurück in einen Optikkanal oder aber gemäß (a) oder (b) in einen zweiten Optikkanal geleitet.

EP 423 367 A zeigt eine gebogene Fiberoptik, wobei zwei Abschnitte von dieser entlang zweier benachbarter paralleler Geraden gegenläufig zueinander verlaufen. Auf einer der Geraden ist eine Lücke zwischen der Fiberoptik ausgebildet, in die ein Medium eindringen kann. Von der Fiberoptik an der Lücke emittiertes Licht muss das in der Lücke befindliche Medium durchdringen und wird anschließend auf der gegenüberliegenden Seite der Lücke von der gebogenen Fiberoptik zu einem Empfänger geleitet. Die Bestimmung von Parametern des Mediums erfolgt somit durch eine Messung von Transmissionsverlusten in der Lücke.

US 5 678 751 A offenbart zwei parallel zueinander angeordnete Fiberoptiken, die in eine Spitze eingebettet sind. Gemeinsam mit dieser Spitze bilden die Fiberoptiken eine ebene Oberfläche aus. Insbesondere wird ein Verfahren beschrieben, das sich mit der Einbettung der Fiberoptiken in die Spitze auseinandersetzt.

Eine weitere mögliche Lösung offenbart die Schweizer Patentanmeldung 01635-05. Die dort vorgestellte Vorrichtung hat zum Gegenstand eine Sonde zur gleichzeitigen Messung von Streulicht und Lichtabsorption bei einer Wellenlänge, wobei ein erstes Gehäuse mit Fenstern für den Durchtritt von Transmissionslicht bzw. Streulicht vorgesehen ist. Weiterhin ist ein zweites, an einem Ende verschlossenes Gehäuse, mit einer Aussparung zur Aufnahme von optischen Elementen für die Führung und Bündelung von Strahlung und mit einem Fenster für den Durchtritt von Strahlung vorhanden. Beide Gehäuse sind mit ihren Fenstern gegenüberliegend angeordnet und dabei über einen Abstandshalter miteinander verbunden.

Nachteilig ist bei dieser Lösung jedoch, dass die notwendigen Linsen innerhalb der beiden Gehäuse gegen Kerosin unzureichend abgedichtet werden können. Die Verklebungen der Linsen gegenüber den Gehäusen werden im Dauerbetrieb häufig undicht, was zu einer Zerstörung der Sensoranordnung führt. Weiterhin nachteilig ist, dass keine Selbstüberprüfung durchgeführt werden kann.

Es ist daher Aufgabe, eine Sensoranordnung zu finden, mit der man kontinuierlich, quantitativ ermittelbar suspendiertes freies Wasser in Treibstoffen sowie die Anwesenheit von reinem Wasser in Behältern bestimmen kann. Dabei muss die Anordnung wartungsfreundlich, dauerhaft ausgebildet sein und zuverlässig funktionieren. Eine kostengünstige Herstellung ist zudem wünschenswert.

Hauptmerkmale der Erfindung sind im kennzeichnenden Teil von Anspruch 1 angegeben. Ausgestaltungen sind Gegenstand der Ansprüche 2 bis 12.

Eine optische Sensoranordnung zur Detektion eines ersten flüssigen Mediums in einem zweiten flüssigen Medium mittels Reflexion eines emittierten Lichtstrahls einer Wellenlänge an einer Reflexionsfläche, mit einer Lichtquelle sowie einem zugehörigen ersten Empfänger hat zwei parallel zueinander angeordnete Glasstablinsen, die in Bezug zu ihrer optischen Achse jeweils eine anpolierte, schräge Grenzfläche aufweisen, wobei
- die Reflexionsfläche gegenüber den zwei Glasstablinsen angeordnet ist, wobei
- die Lichtquelle der ersten Glasstablinse zugeordnet ist, wobei
- der erste Empfänger der zweiten Glasstablinse zugeordnet ist, und wobei
- die schrägen Grenzflächen derart ausgestaltet sind, dass bei alleiniger Anwesenheit des zweiten flüssigen Mediums in einem Detektionsraum der emittierte Lichtstrahl nach Auftreffen auf die Reflexionsfläche zurück in die erste Glasstablinse reflektiert wird, und schon bei sehr niedriger Konzentration des ersten Mediums im zweiten Medium, Anteile von an Tröpfchen des ersten Mediums entstehendem Streulicht über die zweite Glasstablinse zum ersten Empfänger geleitet werden.

Die erfindungsgemäße Ausgestaltung der Sensoranordnung mit zwei Glasstablinsen hat eine Reihe von Vorteilen. Gegenüber der Verwendung von Glasfasern und herkömmlichen Linsen hat die Verwendung der Glasstablinsen (Glasstäbe) den Vorteil, dass der Strahlengang vorhersehbar und damit berechenbar wird. Die im bisherigen Stand der Technik verwendeten Glasfasern werden von Hand gebündelt und hergestellt. Daher war vor der Erfindung jede optische Sensoranordnung in ihrem Strahlengang und ihrem Brechungsindex unterschiedlich. Die sich an die Sensoranordnung anschließende Elektronik musste damit immer auf die jeweiligen Glasfaserbündel kalibriert werden. Eine Serienfertigung der Sensoranordnung war damit faktisch unmöglich. Durch die Verwendung der Glasstablinsen und den dadurch möglichen Verzicht auf Glasfaserbündel und herkömmliche Linsen entsteht ein annähernd paralleles Lichtbündel, die Intensität des Lichts, dass in die flüssigen Medien eintritt wird dadurch eklatant (Faktor 10 bis 100) verbessert. Dadurch werden die Abweichungen in den Genauigkeiten der Sensoren erheblich verringert und es können mittels einer später anschließenden Elektronik somit deutlich mehr und genauere Werte ausgelesen werden. Weiterhin muss die anschließende Elektronik nicht mehr auf jede Sensoreinheit eingemessen werden, sondern kann vielmehr direkt mit dem berechneten Strahlengang programmiert werden. Weiterhin entstehen durch die erfindungsgemäße Ausgestaltung große Vorteile in Bezug auf die Dauerhaftigkeit der Sensoranordnung. Vor der Erfindung gelangte häufig Kerosin zwischen die herkömmlichen Linsen und die Glasfaserbündel, da die eingeklebten Linsen den Druckwechselbeanspruchungen von bis zu 16-bar nicht standhalten konnten. Der optische Pfad war somit zerstört. Durch die Verwendung von Glasstäben als Glasstablinsen kann die Elektronik nun besser vor dem Kerosin geschützt werden, da die Glasstäbe auf Ihrer gesamten Länge gegenüber ihrer Halterung abgedichtet werden können.

Besonders vorteilhaft ist die Ausgestaltung derart, dass die Glasstablinsen einen anderen optischen Brechungsindex als die flüssigen Medien aufweisen. Der Brechungsindex der Glasstäbe ist dabei einheitlich festgelegt, was durch den Einsatz von Spezialglas gewährleistet wird. Das eingeleitete Licht wird beim Übergang von Glas auf Flüssigkeit, bedingt durch die unterschiedlichen Brechungsindizes anders gebrochen und in einem definierten Winkel zur optischen Achse der Glasstablinsen abgelenkt. Die Glasstäbe sind beide im Winkel anpoliert, da sonst der erwünschte Streuwinkel von 20°, trotz Verwendung eines speziellen Glases, nicht erreicht worden wäre. Die anpolierten Flächen sind als schräge Grenzflächen so ausgebildet, dass bei Kerosin der Strahlengang beim Verlassen der Glasstablinsen eine Reflexionsfläche trifft. Der Strahlengang wird an dieser reflektiert und läuft zurück in den gleichen Glasstab (Glasstablinse). Da Wasser einen anderen Brechungsindex als Kerosin besitzt, wird das Licht stärker abgelenkt und trifft die erwähnte Reflexionsfläche nicht. Eine Rückreflexion findet nicht mehr statt. Gegenüber dem Stand der Technik hat diese Ausgestaltung den weiteren Vorteil, dass nur noch eine Lichtquelle mit einer definierten Wellenlänge benötigt wird, da der Absorptionsanteil für die Detektion von Wasser nicht mehr ermittelt werden muss.

Vorzugsweise sind die Glasstablinsen rund ausgeführt. Sie lassen sich damit in einer Halterung um ihre eigene Achse rotieren, was eine nachträgliche Justierung des Reflexionsgangs ermöglicht. Eine Sensoranordnung, bei der die Glasstablinsen in einem Gehäuse gekapselt sind, weist zudem den Vorteil auf, dass die Lichtquelle sowie der zugehörige Empfänger dauerhaft gegenüber dem von Kerosin umspülten Teil abgedichtet werden kann. In einer besonders vorteilhaften Ausgestaltung können die Glasstablinsen mit dem Gehäuse vergossen sein. Die Abdichtung zwischen Gehäuse und den Glasstablinsen ist dann besonders dauerhaft. Als Gehäuse bietet sich neben einem Edelstahlkörper auch ein Spritzgussgrundkörper aus Kunststoff an. Bei einem Spritzgussgrundkörper können die Glasstablinsen mit in diesen eingegossen werden. Die führt neben den erwähnten Vorteilen bei der Abdichtung auch zu einer kostengünstigen und kompakten Bauweise.

Eine erfindungsgemäße Sensoranordnung, bei der die Reflexionsfläche gegenüber den Glasstablinsen angeordnet und mit dem Gehäuse der Glasstablinsen verbunden ist, hat zudem den Vorteil, dass eine kompakte Einheit entsteht. So kann beispielsweise bei der Wartung der Sensoranordnung und beim Ausbau des die Glasstablinsen tragenden Gehäuses zugleich die Reflexionsfläche mit gewartet werden. Dies ist insbesondere von Vorteil, da sich Schwebestoffe auf diesen Reflexionsflächen absetzen können und somit den Reflexionsgrad vermindern. Zusätzlich kann auf der Reflexionsfläche ein zusätzlicher Spiegel zur Verstärkung angebracht sein. Dieser kann so ebenfalls gereinigt werden.

Eine vor der Lichtquelle angeordnete Linse bündelt das emittierte Licht und sorgt für eine ausgewogene Lichtverteilung innerhalb der Glasstablinse. Eine vor dem Empfänger angeordnete Blende begrenzt den auf den Empfänger treffenden Reflexionsstrahl auf ein definiertes Maß an Strahlungsintensität. Weiterhin kann zwischen der Glasstablinse eine weitere Linse angeordnet sein, welche den Strahlgang konzentriert auf den Empfänger lenkt.

Eine besonders vorteilhafte Ausgestaltung der Sensoranordnung sieht vor, dass eine Kontrolleinrichtung der Sende- und Empfangseinheit vorhanden ist. Es kann somit die Funktionsfähigkeit der Sensoranordnung überprüft werden. Weiterhin kann ein Verschmutzungsgrad der Reflexionsfläche ermittelt werden. Auch bietet diese Kontrolleinrichtung die Möglichkeit, einen Wasserschlag zu detektieren, ohne sich einer Absorptionsmessung bedienen zu müssen. Bevorzugt weist die Kontrolleinrichtung einen Strahlteiler sowie einen zweiten Empfänger auf. Der Strahlteiler zweigt dabei einen kleinen Teil des von der Lichtquelle emittierten Lichts ab und reflektiert diesen auf den zweiten Empfänger. Dadurch kann geprüft werden, ob die Lichtquelle überhaupt funktioniert und auch welche Lichtmenge sie emittiert.

Weiterhin ist bevorzugt ein dritter Empfänger an der Sensoranordnung vorgesehen, der dann gegenüber dem zweiten Empfänger angeordnet ist. Der an der Reflexionsfläche reflektierte Lichtstrahl trifft auf den Strahlteiler, der einen kleinen Anteil dessen auf den dritten Empfänger leitet, der die ankommende Lichtintensität so bestimmt. Aus dem Verhältnis der emittierten Lichtmenge zur ankommenden Lichtmenge können verschieden Rückschlüsse getroffen werden. Beispielhaft seien genannt: Grad der Verschmutzung des Spiegels, Grad der Verschmutzung der Grenzflächen, Vorhandensein des Spiegels und eventuell der Grad des Absorptionsanteils durch Wasser in der Kerosinphase. Die gegenüberliegende Anordnung der beiden Empfänger ermöglicht eine kompakte Bauweise.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine erfindungsgemäße Sensoranordnung;
- Fig. 2: Strahlengang bei der Detektion von reinem Kerosin;
- Fig. 3: Strahlengang bei der Detektion von Kerosin-Wasser-Emulsion;
- Fig. 4: Strahlengang bei der Detektion von reinem Kerosin in einer erfindungsgemäßen Sensoranordnung mit Kontrolleinrichtung

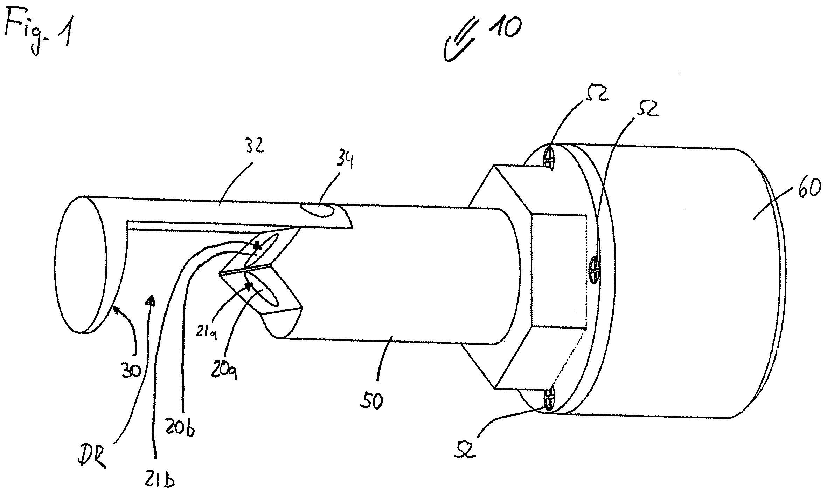

Figur 1 zeigt eine erfindungsgemäße Sensoranordnung 10 im endmontierten Zustand vor deren Einbau in einen Tank oder in eine Rohrleitung. Man erkennt, dass eine erste Glasstablinse 20a sowie eine zweite Glasstablinse 20b in einem Gehäuse 50 vollständig gekapselt sind. Das Gehäuse 50 ist dabei an die Grenzflächen 21a, 21b der Glasstablinsen angepasst. Ein Kragarm 32, mit einer an diesem angeordneten Reflexionsfläche 30, ist über Sicherungsmittel 34 (z. B. Schrauben) mit dem Gehäuse 50 verbunden. Das Gehäuse 50 ist dabei über Sicherungsmittel 52 lösbar mit einem Elektronikgehäuse 60 verbunden. Die Lichtquelle 5 sowie der Empfänger 6 sind dabei in dem Elektronikgehäuse 60 gelagert. Durch diese Ausgestaltung sind alle wesentlichen Elemente der Erfindung leicht zu demontieren und einzeln zu warten. Zwischen der Reflexionsfläche 30 und dem Gehäuse 50 ist ein Detektionsraum DR definiert. Die Wasserkonzentration wird in diesem Detektionsraum DR, dessen Volumen bekannt ist, ermittelt. Mittels Korrelation kann die so gewonnene Aussage über die Wasserkonzentration im Kerosin auf den Tank oder die Rohrleitung hochgerechnet werden. Die Sensoranordnung ist dadurch besonders kompakt ausgebildet.

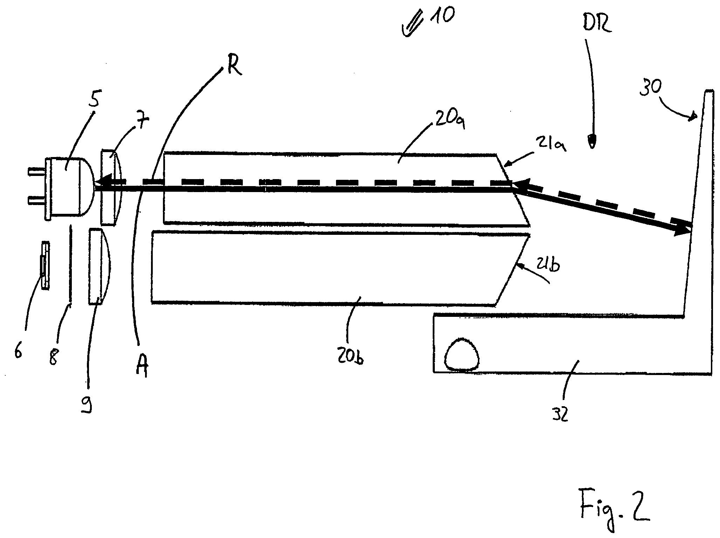

Figur 2 zeigt das Wirkprinzip der erfindungsgemäßen Sensoranordnung 10 bei der Detektion von reinem Kerosin. Eine Lichtquelle 5 emittiert dabei eine Lichtstrahlung A, die durch eine herkömmliche Linse 7 gebündelt in die erste Glasstablinse 20a geschickt wird. Die Glasstablinse 20a richtet dabei die Lichtstrahlung A. An der Grenzfläche 21a, dort wo die Lichtstrahlung A die Glasstablinse 20a verlässt, wird die Lichtstrahlung A gebrochen und trifft auf eine gegenüberliegende Reflexionsfläche 30. An dieser Reflexionsfläche 30 können reflexionssteigernde Beschichtungen (Verspiegelungen) angebracht sein. Der an der Reflexionsfläche 30 reflektierte Lichtstrahl R (Reflexionsstrahl) wird wieder in die erste Glasstablinse 20a geführt. An deren Ende kann sich ein zusätzlicher (nicht dargestellter) Empfänger befinden. Die Wellenlänge des emittierten Lichts ist so gewählt, dass bei reinem Kerosin im Detektionsraum DR die Streulichtstrahlung annähernd absorbiert wird. Die zweite Glasstablinse 20b leitet dann wenig bis keine Strahlung durch die Linse 9 und die Blende 8 auf den Streulichtempfänger 6.

Bei reinem Wasser im Detektionsraum DR wird die Lichtstrahlung A so stark abgelenkt, dass diese nicht mehr auf die erste Glasstablinse 20a zurückreflektiert wird. Der Streulichtanteil, wäre allerdings merklich höher, da die Absorption durch das fehlende Kerosin geringer ausfallen würde. Der Streulichtanteil würde allerdings durch die zweite Glasstablinse 20b geführt.

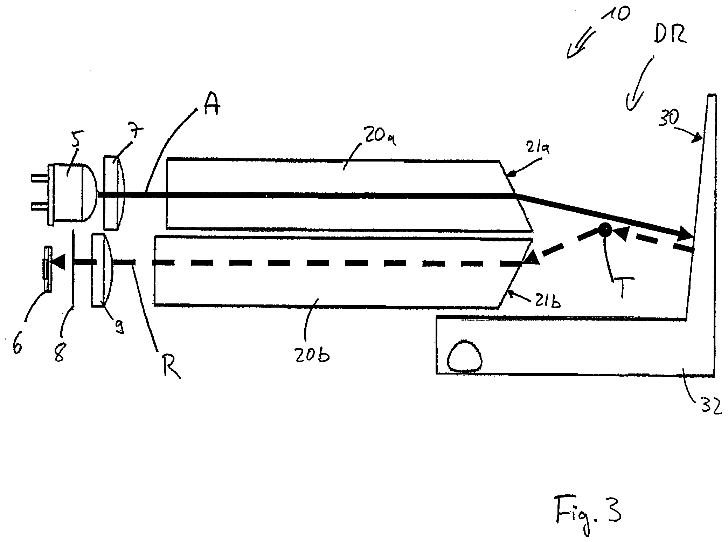

Figur 3 zeigt das Wirkprinzip der erfindungsgemäßen Sensoranordnung 10 bei Vorhandensein von Wassertröpfchen T. Bei vorhandenen Wassertröpfchen T entsteht Streulicht, welches als Vorwärts- und Rückwärtsstreuung detektiert werden kann. Bei einer erfindungsgemäßen Ausgestaltung mit Reflexionsfläche 30 werden beide Effekte genutzt. Insbesondere die dargestellte Vorwärtsstreuung ist von Interesse. Der emittierte Lichtstrahl A wird an der Reflexionsfläche 30 reflektiert und trifft anschließend die Wassertröpfchen T. Das Streulicht wird über die zweite Glasstablinse 20b, durch die Linse 9 und die Blende 8 auf den Empfänger 6, der die Streulichtstrahlung detektiert, geleitet. Die Empfindlichkeit des Empfängers 6 ist dabei so ausgebildet, dass selbst sehr niedrige Wasserkonzentrationen (bis zu 5 PPM Wasser in Kerosin) ermittelt werden. Je mehr Wasser vorhanden ist, desto höher mehr Streuungslicht gelangt in die zweite Glasstablinse 20b.

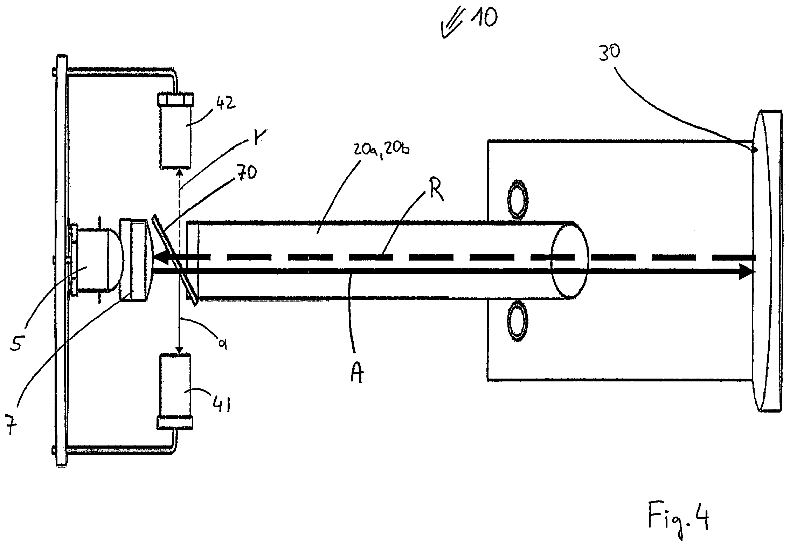

Figur 4 zeigt bei einer besonders vorteilhaft ausgebildeten erfindungsgemäßen Sensoranordnung 10 mit Kontrolleinrichtung den Strahlengang bei der Detektion von reinem Kerosin. Die Darstellung der Sensoranordnung 10 ist gegenüber Figur 2 um 90 Grad gedreht. Die Lichtstrahlung A verlässt die Lichtquelle 5 durch die Linse 7 und trifft dort auf einen Strahlteiler 70. Die Lichtstrahlung A passiert den Strahlteiler 70, der einen Teil des Lichtes a auf einen zweiten Empfänger 41 schickt. Der zweite Empfänger 41 ist dabei eine Referenzmesszelle, vorzugsweise eine Photozelle. Dadurch ist es möglich, die Sendeleistung der Lichtquelle 5, und damit auch die in die erste Glasstablinse 20a eintretende Lichtstrahlung, zu messen. Das nicht vom Strahlteiler 70 auf den zweiten Empfänger 41 abgeteilte Licht geht durch die erste Glasstablinse 20a und wird beim Übergang an der Grenzfläche 21a gebrochen. Bei Kerosin erreicht die Lichtstrahlung A die Reflexionsfläche, wird an dieser reflektiert und gelangt durch die erste Glasstablinse 20a zurück zum Strahlteiler 70. Der Strahlteiler 70 leitet einen Anteil r der Reflexionsstrahlung R auf einen dritten Empfänger 42. Durch die Messung dieses Anteils r können die Verschmutzungsgrade der Reflexionsfläche 30 sowie der Grenzfläche 21a ermittelt werden. Bevorzugt sind die vom Strahlungsteiler 70 abgetrennten Strahlungsanteile (r,a) gleich groß in Bezug auf die Lichtstrahlungen (R, A).

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

**Bezugszeichenliste**

| | | | |
|---|---|---|---|
| 5 | Lichtquelle | A | Lichtstrahlung |
| 6 | Empfänger | R | Refelxsionsstrahlung |
| 7 | herkömmliche Linse | DR | Detektionsraum |
| 8 | Blende | T | Wassertröpfchen |
| 9 | herkömmliche Linse | | |
| | | | |
| 10 | Sensoranordnung | | |
| | | | |
| 20 | Glasstablinsen | | |
| 20a | erste Glasstablinse | | |
| 20b | zweite Glasstablinse | | |
| 21a | erste Grenzfläche | | |
| 21b | zweite Grenzfläche | | |
| | | | |
| 30 | Reflexionsfläche | | |
| 32 | Kragarm | | |
| 34 | Sicherungsmittel | | |
| | | | |
| 41 | zweiter Empfänger | | |
| 42 | dritter Empfänger | | |
| | | | |
| 50 | Gehäuse | | |
| 52 | Sicherungsmittel | | |
| | | | |
| 60 | Elektronikgehäuse | | |
| | | | |
| 70 | Strahlteiler | | |

## Patentansprüche

1. Optische Sensoranordnung (10) zur Detektion eines ersten flüssigen Mediums in einem zweiten flüssigen Medium mittels Reflexion eines emittierten Lichtstrahls (A) einer Wellenlänge an einer Reflexionsfläche (30), mit einer Lichtquelle (5) sowie einem zugehörigen ersten Empfänger (6), mit
- zwei Glasstablinsen (20a,20b) die parallel zueinander angeordnet sind, **dadurch gekennzeichnet, dass** die Glasstablinsen in Bezug zu ihrer optischen Achse jeweils eine anpolierte, schräge Grenzfläche (21a,21b) aufweisen,
- die Reflexionsfläche (30) gegenüber den zwei Glasstablinsen (20a,20b) angeordnet ist,
- die Lichtquelle (5) der ersten Glasstablinse (20a) zugeordnet ist,
- der erste Empfänger (6) der zweiten Glasstablinse (20b) zugeordnet ist,
- die schrägen Grenzflächen (21a,21b) derart ausgestaltet sind, dass bei alleiniger Anwesenheit des zweiten flüssigen Mediums in einem Detektionsraum (DR) der emittierte Lichtstrahl (A) nach Auftreffen auf die Reflexionsfläche (30) zurück in die erste Glasstablinse (20a) reflektiert wird, und schon bei sehr niedriger Konzentration des ersten Mediums im zweiten Medium, Anteile von an Tröpfchen des ersten Mediums entstehendem Streulicht über die zweite Glasstablinse (20b) zum ersten Empfänger (6) geleitet werden.

2. Sensoranordnung (10) nach Anspruch 1, **dadurch gekennzeichnet, dass** die Glasstablinsen (20) einen anderen optischen Brechungsindex als die flüssigen Medien aufweisen.

3. Sensoranordnung (10) nach einem der vorhergehenden Ansprüche, **dadurch gekennzeichnet, dass** die Glasstablinsen (20) rund sind.

4. Sensoranordnung (10) nach einem der vorhergehenden Ansprüche, **dadurch gekennzeichnet, dass** die Glasstablinsen in einem Gehäuse (50) gekapselt sind.

5. Sensoranordnung (10) nach Anspruch 4, **dadurch gekennzeichnet, dass** die Reflexionsfläche (30) mit dem Gehäuse (50) verbunden ist.

6. Sensoranordnung (10) nach einem der vorhergehenden Ansprüche, **dadurch gekennzeichnet, dass** vor der Lichtquelle (5) eine Linse (7) angeordnet ist.

7. Sensoranordnung (10) nach einem der vorhergehenden Ansprüche, **dadurch gekennzeichnet, dass** vor dem Empfänger (6) eine Blende (8) angeordnet ist.

8. Sensoranordnung (10) nach Anspruch 7, **dadurch gekennzeichnet, dass** vor der Blende (8) eine weitere Linse (9) angeordnet ist.

9. Sensoranordnung (10) nach einem der vorhergehenden Ansprüche, **dadurch gekennzeichnet, dass** eine Kontrolleinrichtung einer Sende- und Empfangseinheit vorhanden ist.

10. Sensoranordnung (10) nach Anspruch 9, **dadurch gekennzeichnet, dass** die Kontrolleinrichtung einen Strahlteiler (70) sowie einen zweiten Empfänger (41) aufweist.

11. Sensoranordnung (10) nach Anspruch 10, **dadurch gekennzeichnet, dass** die Kontrolleinrichtung einen dritten Empfänger (42) aufweist.

12. Sensoranordnung (10) nach Anspruch 11, **dadurch gekennzeichnet, dass** der zweite Empfänger (41) und der dritte Empfänger (42) einander gegenüber angeordnet sind.

## Claims

1. Optical sensor arrangement (10) for detection of a first liquid medium in a second liquid medium by means of reflection of an emitted light beam (A) of a wavelength at a reflective surface (30), having a light source (5) as well as an associated first receiver (6), having
- two glass rod lenses (20a, 20b) which are arranged in parallel to each other, **characterised in that** the glass rod lenses each have a to some degree polished, sloping boundary surface (21a, 21b) with regard to their optical axis respectively,
- the reflective surface (30) is arranged opposite the two glass rod lenses (20a, 20b),
- the light source (5) is allocated to the first glass rod lens (20a),
- the first receiver (6) is allocated to the second glass rod lens (20b),
- the sloping boundary surfaces (21a, 21b) are designed in such a way that in the case of sole presence of the second liquid medium in a detection chamber (DR), the emitted light beam (A) is reflected back into the first glass rod lens (20a) after impact on the reflective surface (30), and yet in the case of a very low concentration of the first medium in the second medium, portions of scattered light resulting from drops of the first medium are led to the first receiver (6) via the second glass rod lens (20b).

2. Sensor arrangement (10) according to claim 1, **characterised in that** the glass rod lenses (20) have a different optical refractive index to the liquid media.

3. Sensor arrangement (10) according to one of the preceding claims, **characterised in that** the glass rod lenses (20) are round.

4. Sensor arrangement (10) according to one of the preceding claims, **characterised in that** the glass rod lenses are encapsulated in a housing (50).

5. Sensor arrangement (10) according to claim 4, **characterised in that** the reflective surface (30) is connected to the housing (50).

6. Sensor arrangement (10) according to one of the preceding claims, **characterised in that** a lens (7) is arranged in front of the light source (5).

7. Sensor arrangement (10) according to one of the preceding claims, **characterised in that** an aperture (8) is arranged in front of the receiver (6).

8. Sensor arrangement (10) according to claim 7, **characterised in that** a further lens (9) is arranged in front of the aperture (8).

9. Sensor arrangement (10) according to one of the preceding claims, **characterised in that** a control device of a transmitting and receiving unit is present.

10. Sensor arrangement (10) according to claim 9, **characterised in that** the control device has a beam splitter (70) as well as a second receiver (41).

11. Sensor arrangement (10) according to claim 10, **characterised in that** the control device has a third receiver (42).

12. Sensor arrangement (10) according to claim 11, **characterised in that** the second receiver (41) and the third receiver (42) are arranged opposite each other.

## Revendications

1. Dispositif capteur optique (10) destiné à la détection d'un premier fluide dans un deuxième fluide au moyen de la réflexion d'un faisceau lumineux émis (A) d'une longueur d'onde au niveau d'une surface de réflexion (30), avec une source de lumière (5) etun premier récepteur (6) associé,
- avec deux lentilles tiges de verre (20a, 20b) qui sont agencées parallèlement l'une à l'autre, **caractérisé en ce que** les lentilles tiges de verre présentent par rapport à leur axe optique à chaque fois une surface limite oblique (21a, 21b) polie dans une certaine mesure,
- **en ce que** la surface de réflexion (30) est agencée en face des deux lentilles tiges de verre (20a, 20b),
- **en ce que** la source de lumière (5) est associée à la première lentille tigede verre (20a),
- **en ce que** le premier récepteur (6) est associé à la deuxième lentille tigede verre (20b), **en ce que** les surfaces limites obliques (21a, 21b) sont conçues de telle sorte que, lors de la seule présence du deuxième fluide dans un espace de détection (DR), le faisceau lumineux émis (A) est réfléchi après son incidence sur la surface de réflexion (30) en direction de la première lentille tigede verre (20a) et, même avec une très faible concentration du premier fluide dans le deuxième fluide, des parties de lumière diffusée créée au niveau de gouttelettes du premier fluide sont conduites au premier récepteur (6) par l'intermédiaire de la deuxième lentille tige de verre (20b).

2. Dispositif capteur (10) selon la revendication 1, **caractérisé en ce que** les lentilles tiges de verre (20) présentent un indice de réfraction optique différent de celui des fluides.

3. Dispositif capteur (10) selon l'une des revendications précédentes, **caractérisé en ce que** les lentilles tiges de verre (20) sont rondes.

4. Dispositif capteur (10) selon l'une des revendications précédentes, **caractérisé en ce que** les lentilles tigesde verre sont encapsulées dans un boîtier (50).

5. Dispositif capteur (10) selon la revendication 4, **caractérisé en ce que** la surface de réflexion (30) est assemblée au boîtier (50).

6. Dispositif capteur (10) selon l'une des revendications précédentes, **caractérisé en ce qu'**une lentille (7) est agencée devant la source de lumière (5).

7. Dispositif capteur (10) selon l'une des revendications précédentes, **caractérisé en ce qu'**un diaphragme (8) est agencé devant le récepteur (6).

8. Dispositif capteur (10) selon la revendication 7, **caractérisé en ce qu'**une autre lentille (9) est agencée devant le diaphragme (8).

9. Dispositif capteur (10) selon l'une des revendications précédentes, **caractérisé en ce qu'**un dispositif de contrôle d'une unité d'émission et de réception est présent.

10. Dispositif capteur (10) selon la revendication 9, **caractérisé en ce que** le dispositif de contrôle comporte un séparateur de faisceau (70) ainsi qu'un deuxième récepteur (41).

11. Dispositif capteur (10) selon la revendication 10, **caractérisé en ce que** le dispositif de contrôle comporte un troisième récepteur (42).

12. Dispositif capteur (10) selon la revendication 11, **caractérisé en ce que** le deuxième récepteur (41) et le troisième récepteur (42) sont agencés en face l'un de l'autre.
